# EUROPEAN PATENT APPLICATION

(11) **EP 0 628 567 A1**
(43) Date of publication of application: **14.12.1994**
(21) Application number: 93922040.6
(22) Date of filing: 06.10.1993
(51) Int. Cl.: C07H 19/048, C07D 401/04

(54) **NICOTINE DERIVATIVE**

(30) Priority: 07.10.1992 JP 268821/92; 17.03.1993 JP 57476/93; 16.06.1993 JP 145077/93; 22.09.1993 JP 236067/93
(71) Applicant: JAPAN TOBACCO INC., Shinagawa-ku, Tokyo 140 (JP)
(72) Inventor: SHIBAGAKI, Makoto, Japan Tobacco Inc., Yokohama-shi, Kanagawa-ken 227 (JP); TAKAHASHI, Kyoko, Japan Tobacco Inc., Yokohama-shi, Kanagawa-ken 227 (JP); MATSUSHITA, Hajime, Japan Tobacco Inc., Yokohama-shi, Kanagawa-ken 227 (JP); KUNO, Hideyuki, Japan Tobacco Inc., Yokohama-shi, Kanagawa-ken 227 (JP)
(74) Representative: Ruffles, Graham Keith
(86) International application number: JP9301434
(87) International publication number: WO9407903

(57) **Abstract**

A novel sugar-containing nicotine derivative represented by general formula (1), wherein R represents a ribofuranosyl group; and X represents an oxygen atom or two hydrogen atoms. This compound is an excellent smoking taste modifier and has the effects of restoring the odor and taste of tobacco lost in the production of shredded tobacco and imparting a fine pattern of spreading to the smoke. Because the compound (1) has a characteristic structure containing ribofuranose in its molecule and also containing a physiologically active nicotine as the aglycon, it is expected to be used as a medicine with a reduced toxicity besides the smoking taste modifier. The invention also provides another novel nicotine derivative represented by general formula (2) wherein X is as defined above.

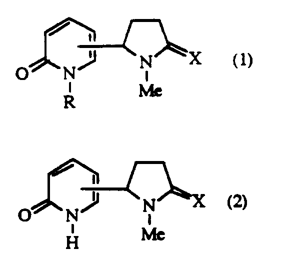

## Description

### Technical Field

The present invention relates to a novel and optically active nicotine derivative, more particularly to an optically active nicotine derivative containing a glycosyl, represented by the following formula (1): 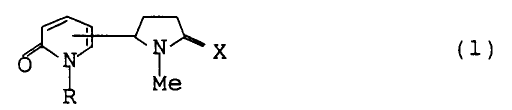
wherein R is a ribofuranosyl group, and X is an oxygen atom or two hydrogen atoms.

Further, the present invention relates to a novel nicotine derivative represented by the following formula (2): 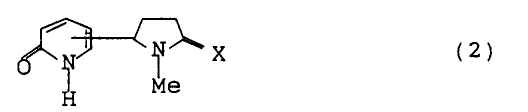
wherein X is the same as defined above.

### Background Art

Nicotine is an alkaloid obtained as a by-product in the tobacco industry. A useful derivative of nicotine is also known to have an effect of improving the flavor and taste of tobacco (for example, Jpn. Pat. Nos. 1371806 and 1456909).

Well-known physiological activities of nicotine include an autonomic nervous regulatory activity, a circulatory-system regulatory activity such as vassopressor activity, and a digestive organ regulatory activity such as gastrointestinal activation (for example, Seikagaku, 57, 447 (1985), J. Pharm. Experi, Ther., 222, 463 (1983), Br. J. Pharmac., 79, 869 (1983)). Further, alkoxy nicotine is known to have an antimicrobial activity and an antibacterial activity (U.S. Patent Serial No. 3,644,176). As mentioned above, nicotine and a nicotine derivative have a variety of physiological activities, and they are expected to be useful as a pharmaceutical agent. In addition, a sulfate of nicotine is used as an agricultural insecticide, and its application to the agricultural field is also expected.

However, since nicotine has toxicity by itself and its volatility is high, application of nicotine to the above-mentioned individual fields or practical use of nicotine in those field has not yet been achieved. Accordingly, there is a request for a nicotine derivative having useful properties and capable of solving a problem such as toxicity.

The present invention has been made under the above-mentioned circumstances and has an object to provide an optically active novel nicotine derivative containing a glycosyl and a novel nicotine derivative which are useful as an agent for improving the flavor and taste of tobacco, and which can be expected to be used as a physiologically active substance as mentioned above.

### Disclosure of Invention

It is a first object of the present invention to provide a novel and optically active nicotine derivative containing a glycosyl group.

It is a second object of the present invention to provide a novel nicotine derivative.

In short, the present invention relates to a novel nicotine derivative containing a glycosyl group represented by the following formula (1): 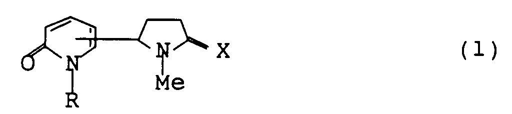
wherein R and X are the same as defined above.

Further, the present invention relates to a novel nicotine derivative represented by the following formula (2): 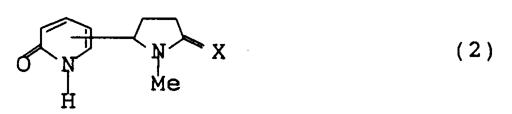
wherein X is the same as defined above.

Hereinbelow, the present invention will be explained in detail.

The novel nicotine derivative containing a glycosyl group according to the first aspect of the present invention is a compound represented by the following formula (1): 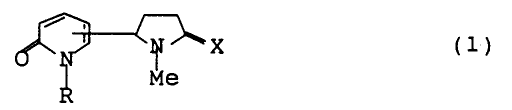
wherein, R and X are the same as defined above.

In the compound (1) of the present invention, R is a ribofuranosyl group since ribose is easy to obtain, inexpensive, and relatively easier to be converted into a glycosyl donor.

In the compound (1) of the present invention, an aglycone is a group represented by the following formula (3). As long as it is the group represented by the formula (3), the aglycone is not particularly limited. 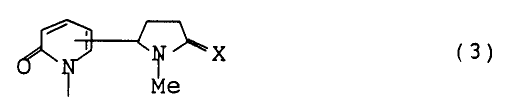
wherein X is the same as defined above.

In the present invention, as a group represented by the formula (3), a (1-methyl-2-pyrrolidinyl)-2-pyridone group or a (1-methyl-5-oxo-2-pyrrolidinyl)-2-pyridone group is preferable since they are relatively easier to synthesize. An (S)-(1-methyl-2-pyrrolidinyl)-2-pyridone group, an (R)-(1-methyl-2-pyrrolidinyl)-2-pyridone group, an (S)-(1-methyl-5-oxo-pyrrolidinyl)-2-pyridone group, or an (R)-(1-methyl-5-oxo-2-pyrrolidinyl)-2-pyridone group is more preferable. In the present invention, the most preferable group is an (S)-(-)-3-(1-methyl-2-pyrrolidinyl)-2-pyridone group (3a), an (R)-(+)-3-(1-methyl-2-pyrrolidinyl)-2-pyridone group (3b), an (S)-(-)-5-(1-methyl-2-pyrrolidinyl)-2-pyridone group (3c), an (R)-(+)-5-(1-methyl-2-pyrrolidinyl)-2-pyridone group (3d), an (S)-(-)-3-(1-methyl-5-oxo-2-pyrrolidinyl)-2-pyridone group (3e), or an (S)-(-)-5-(1-methyl-5-oxo-2-pyrrolidinyl)-2-pyridone group (3f). 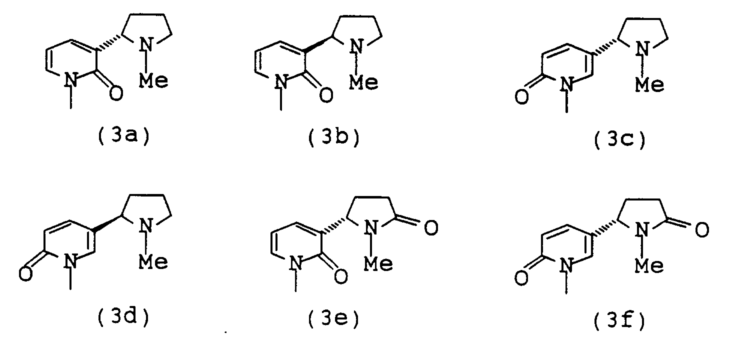
The novel nicotine derivative according to the second aspect of the present invention is a compound represented by a general formula (2): 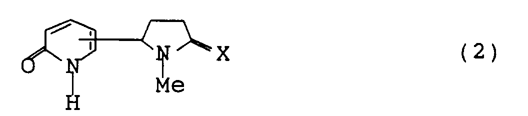
wherein X is the same as defined above.

In the present invention, as the novel nicotine derivative represented by the above-mentioned formula (2), preferably (1-methyl-2-pyrrolidinyl)-2(1H)-pyridone or (1-methyl-5-oxo-2-pyrrolidinyl)-2(1H)-pyridone is used since they are relatively easier to synthesize. More preferably, (S)-(1-methyl-2-pyrrolidinyl)-2(1H)-pyridone, (R)-(1-methyl-2-pyrrolidinyl)-2(1H)-pyridone, (S)-(1-methyl-5-oxo-2-pyrrolidinyl)-2(1H)-pyridone, or (R)-(1-methyl-5-oxo-2-pyrrolidinyl)-2(1H)-pyridone is used. In the present invention, the most preferable novel nicotine derivative is an optically active (S)-(-)-3-(1-methyl-2-pyrrolidinyl)-2(1H)-pyridone (2a), (R)-(+)-3-(1-methyl-2-pyrrolidinyl)-2(1H)-pyridone (2b), (S)-(-)-5-(1-methyl-2-pyrrolidinyl)-2(1H)-pyridone (2c), (R)-(+)-5-(1-methyl-2-pyrrolidinyl)-2(1H)-pyridone (2d), (S)-(-)-3-(1-methyl-5-oxo-2-pyrrolidinyl)-2(1H)-pyridone (2e), or (S)-(-)-5-(1-methyl-5-oxo-2-pyrrolidinyl)-2(1H)-pyridone (2f). 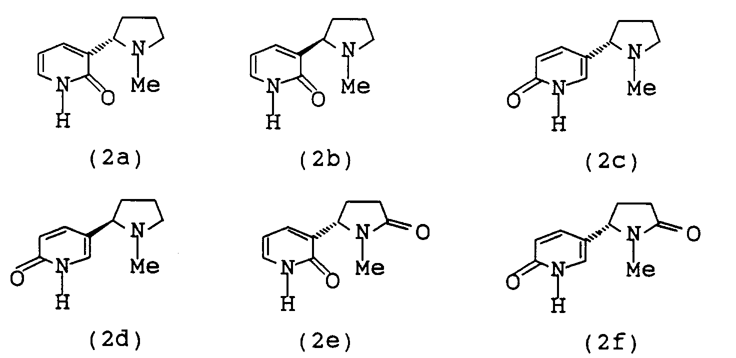
Hereinbelow, the optically active novel nicotine derivative (1) containing a glycosyl group, of the present invention will be described in detail.

First of all, the features of the compound (1) of the present invention will be described.

The compound (1) of the present invention is characterized in that it has a group represented by the formula (3) as an aglycone and a ribofuranosyl group as a glycosyl part, both of which are bound with N-glycosidic bond. A compound having a β-N-glycoside bond is more preferable since it is easy to prepare. In the present invention, a compound represented by the following formula (1') is therefore the most preferable: 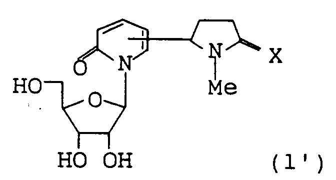
wherein X is the same as defined above.

Further, in the present invention, since the above-mentioned groups (3a) to (3f) are especially preferable as an aglycone, it is preferred that the compounds (1) and (1') are represented by the following 6 formulas (1a) to (1f). 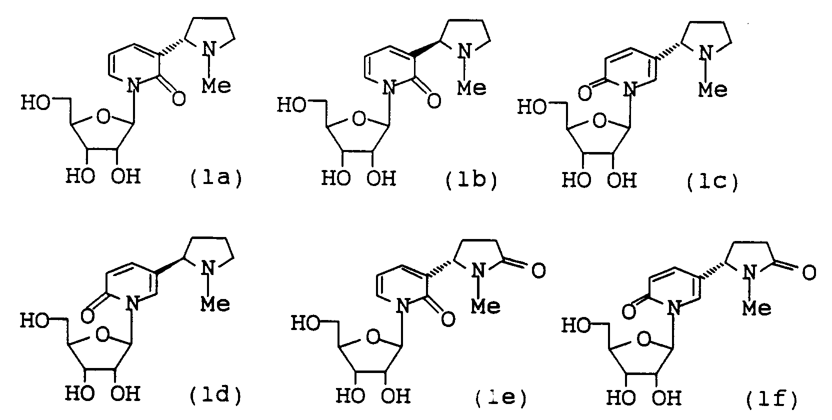
Hereinbelow, the features of especially preferable compounds (1a) to (1f) of the present invention will be described.

### 1. Compound (1a)

The compound (1a) is characterized in that it has an (S)-(-)-3-(1-methyl-2-pyrrolidinyl)-2-pyridone group as an aglycone and a ribofuranosyl group as a glycosyl part, both of which are bound with β-N-glycosidic bond.

### 2. Compound (1b)

The compound (1b) is characterized in that it has an (R)-(+)-3-(1-methyl-2-pyrrolidinyl)-2-pyridone group as an aglycone and a ribofuranosyl group as a glycosyl part, both of which are bound with β-N-glycosidic bond.

### 3. Compound (1c)

The compound (1c) is characterized in that it has an (S)-(-)-5-(1-methyl-2-pyrrolidinyl)-2-pyridone group as an aglycone and a ribofuranosyl group as a glycosyl part, both of which are bound with β-N-glycosidic bond.

### 4. Compound (1d)

The compound (1d) is characterized in that it has an (R)-(+)-5-(1-methyl-2-pyrrolidinyl)-2-pyridone group as an aglycone and a ribofuranosyl group as a glycosyl part, both of which are bound with β-N-glycosidic bond.

### 5. Compound (1e)

The compound (1e) is characterized in that it has an (S)-(-)-3-(1-methyl-5-oxo-2-pyrrolidinyl)-2-pyridone group as an aglycone and a ribofuranosyl group as a glycosyl part, both of which are bound with β-N-glycosidic bond.

### 6. Compound (1f)

The compound (1f) is characterized in that it has an (S)-(-)-5-(1-methyl-5-oxo-2-pyrrolidinyl)-2-pyridone group as an aglycone and a ribofuranosyl group as a glycosyl part, both of which are bound with β-N-glycosidic bond.

Second, a method for producing the compound (1) of the present invention will be described.

The method for producing the compound (1) is not particularly restricted as long as the compound (1) is able to be synthesized with high efficiency. Hereinbelow, for simplicity sake, the method of the present invention will be described with reference to a method for producing the compound (1'), which should not be construed as limiting the scope of the present invention.

The compound (1'), for example, can be synthesized in accordance with the following reactions: 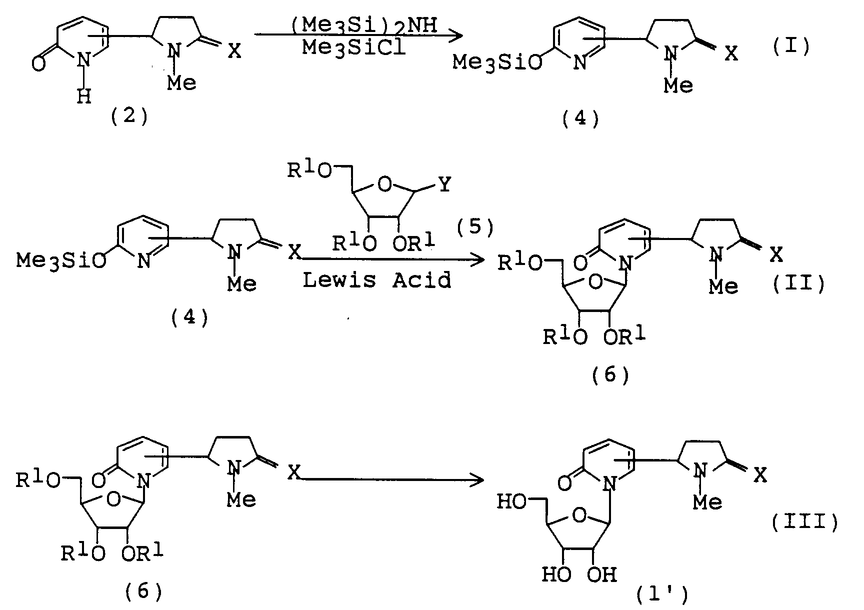
wherein X is the same as defined above; R¹ is a protecting group of a hydroxyl group; and Y is an active group such as an acetyl group.

First, the compound represented by a general formula (4) is prepared from the nicotine derivative (2) of the present invention as shown in a reaction (I). Then, appropriately protected ribofuranose (5) is reacted with the compound represented by the general formula (4) [reaction (II)]. The obtained compound represented by a general formula (6) is deprotected, thereby to synthesize the compound (1') [reaction (III)].

As to a method for producing the nicotine derivative (2) of the present invention, it will be described later.

Hereinbelow, the reactions (I) to (III) will be successively described.

In the reaction (I), the compound (4) can be obtained by refluxing the nicotine derivative (2) of the present invention together with hexamethyldisilazane and trimethylsilyl chloride.

In the reaction (II), the compound (4) obtained in the reaction (I) is reacted with a glycosyl donor, a ribofuranoside derivative (5). To be more specific, the compounds (4) and (5) are stirred in the presence of a Lewis acid in an appropriate solvent.

In this reaction, the ribofuranoside derivative (5) is a glycosyl donor appropriately protected. "The appropriately protected glycosyl donor" is defined as a compound whose hydroxyl groups except a reduced end are protected with an acyl group such as a benzoyl group, or an aralkyl group such as a benzyl group, and whose reduced end is protected with a protecting group which can function as an active group such as an acetyl group. In the present invention, the hydroxyl groups except the reduced end are preferably protected with an acyl group. This is because the 2-, 3-, and 5-positions of the ribofuranosyl group in a reaction (III) to be described later is easily deprotected. Accordingly, as the ribofuranosyl derivative (5), commercially available 1-O-acetyl-2,3,5,-tri-O-benzoyl-β-D-ribofuranose (5a) can be most preferably used in this reaction. 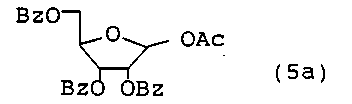
Hereinafter, Bz will denote a benzyl group and Ac will denote an acetyl group.

The solvent to be used in the reaction (II) is not particularly restricted as long as it is used in a general glycosylation reaction. The examples of the solvent include chloroform, methylene chloride, 1,2-dichloroethane, and the like.

The Lewis acid to be used in this reaction is not particularly restricted as long as it promotes N-glycosylation in the reaction (II). The examples of the Lewis acid include tin tetrachloride, titanium tetrachloride, and the like.

The reaction temperature and reaction time can be chosen depending on a reagent to be used in the reaction. The reaction may be preferably performed at about 50°C for approximately 1 to 24 hours.

The obtained product can be purified by means of column chromatography or the like.

In the reaction (III), the nicotine derivative (1') of the present invention containing a glycosyl group is obtained by deprotecting the compound (6) obtained in the reaction (II).

The deprotection is not particularly restricted as long as the protecting groups (R¹) of the 2-, 3-, and 5-positions of the compound (6) obtained in the previous step (II) can efficiently be removed. For example, in the case where R¹ of the compound (6) is an acyl group, the deprotection is performed by treating with a base in an appropriate solvent.

Hereinbelow, the case that R¹ of the compound (6) is an acyl group will be explained.

In the case where R¹ of the compound (6) is an acyl group, the reaction (III) is performed in the presence of a base.

The base to be used in the reaction (III) is not particularly restricted as long as it can remove the protecting group (R¹) of the ribofuranosyl group. The examples of such base include ammonia, sodium methoxide, and the like.

The solvent to be used in the reaction (III) is not particularly restricted as long as it can be used in a general hydrolysis of an acyl group. The examples of such solvent include an alcohol such as methanol.

The temperature and the reaction time can be chosen depending on a reagent to be used in the reaction. For example, the reaction is performed preferably at about 50°C for approximately 30 minutes to 24 hours.

Hereinbelow, the method of producing the compound of the present invention will be more specifically explained with reference to the method for producing particularly preferable compounds (1a) to (1f) of the present invention.

### [A] Synthesis of (S)-(-)-3-(1-methyl-2-pyrrolidinyl)-2-pyridone-N-β-D-ribofuranoside (1a)

(S)-(-)-3-(1-methyl-2-pyrrolidinyl)-2(1H)-pyridone (2a) was refluxing together with hexamethyldisilazane and trimethylsilyl chloride to give (S)-(-)-3(1-methyl-2-pyrrolidinyl)-2-trimethylsiloxy pyridine (4a). This compound (4a) is reacted with 1-O-acetyl-2,3,5-tri-O-benzoyl-β-D-ribofuranose (5a) in the presence of a Lewis acid such as tin tetrachloride or titanium tetrachloride in a solvent such as chloroform or methylene chloride to be used in a general glycosylation, thereby to obtain (S)-(-)-3-(1-methyl-2-pyrrolidinyl)-2-pyridone-2,3,5-tri-O-benzoyl-N-β-D-ribofuranoside (6a). The obtained product can be purified by means of column chromatography.

Then, the obtained product (6a) is treated with a base such as ammonia or sodium methoxide in a solvent such as methanol, thereby to obtain (S)-(-)-3-(1-methyl-2-pyrrolidinyl)-2-pyridone-N-β-D-ribofuranoside (1a). The obtained product can be purified by means of column chromatography. 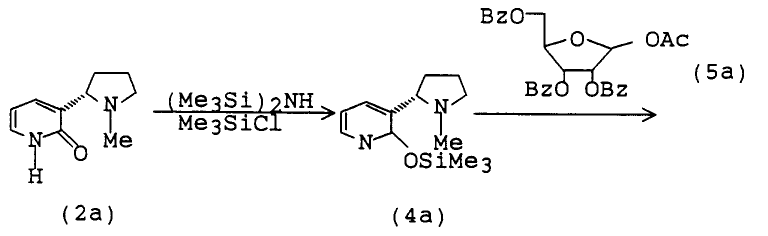 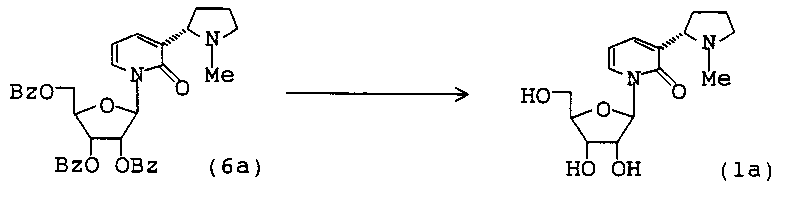
The compound (6a) is characterized in that it has an (S)-(-)-3-(1-methyl-2-pyrrolidinyl)-2-pyridone group as an aglycone and a 2,3,5-tri-O-benzoylribofuranosyl group as a glycosyl part, both of which are bound with β-N-glycosidic bond. The compound (6a) is an important intermediate to prepare the compound (1a) as well as a nicotine derivative having useful properties by itself.

### [B] Synthesis of (R)-(+)-3-(1-methyl-2-pyrrolidinyl)-2-pyridone-N-β-D-ribofuranoside (1b)

As shown in the reaction below, the compound (1b) can be obtained in the same manner as in the above-mentioned [A] except that (R)-(+)-3-(1-methyl-2-pyrrolidinyl)-2(1H)-pyridone (2b) and 1-O-acetyl-2,3,5-tri-O-benzoyl-β-D-ribofuranose (5a) are used. The compounds (1b) and (6b) can be purified by means of column chromatography.

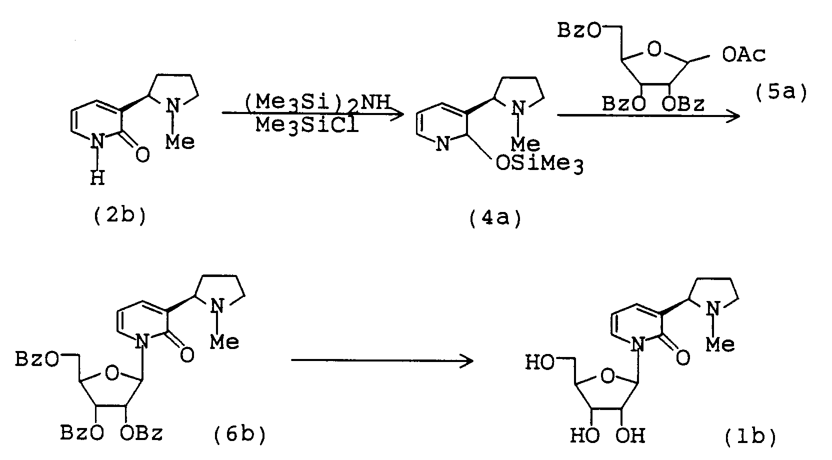

The compound (6b) is characterized in that it has an (R)-(+)-3-(1-methyl-2-pyrrolidinyl)-2-pyridone group as an aglycone and a 2,3,5-tri-O-benzoylribofuranosyl group as a glycosyl part, both of which are bound with β-N-glycosidic bond. The compound (6b) is an important intermediate to prepare the compound (1b) as well as a nicotine derivative having useful properties by itself.

### [C] Synthesis of (S)-(-)-5-(1-methyl-2-pyrrolidinyl)-2-pyridone-N-β-D-ribofuranoside (1c)

As shown in the reaction below, the compound (1c) can be obtained in the same manner as in the step [A] except that (S)-(-)-5-(1-methyl-2-pyrrolidinyl)-2(1H)pyridone (2c) and 1-O-acetyl-2,3,5-tri-O-benzoyl-β-D-ribofuranose (5a) are used. The compounds (1c) and (6c) can be purified by means of column chromatography. 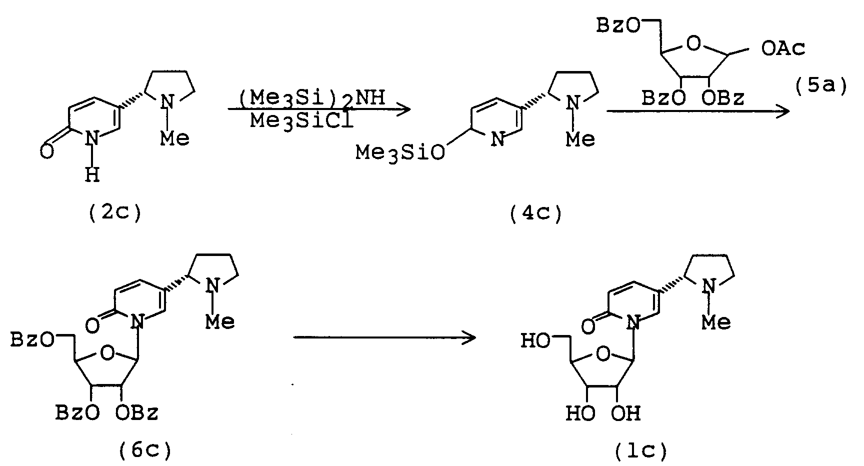
The compound (6c) is characterized in that it has an (S)-(-)-5-(1-methyl-2-pyrrolidinyl)-2-pyridone group as an aglycone and a 2,3,5-tri-O-benzoylribofuranosyl group as a glycosyl part, both of which are bound with β-N-glycosidic bond. The compound (6b) is an important intermediate to prepare the compound (1b) as well as a nicotine derivative having useful properties by itself.

### [D] Synthesis of (R)-(+)-5-(1-methyl-2-pyrrolidinyl)-2-pyridone-N-β-D-ribofuranoside (1d)

As shown in the reaction below, the compound (1d) can be obtained in the same manner as in the step [A] except that (R)-(+)-5-(1-methyl-2-pyrrolidinyl)-2(1H)-pyridone (2d) and 1-O-acetyl-2,3,5-tri-O-benzoyl-β-D-ribofuranose (5a) are used. The compounds (1c) and (6c) can be purified by means of column chromatography and the like. 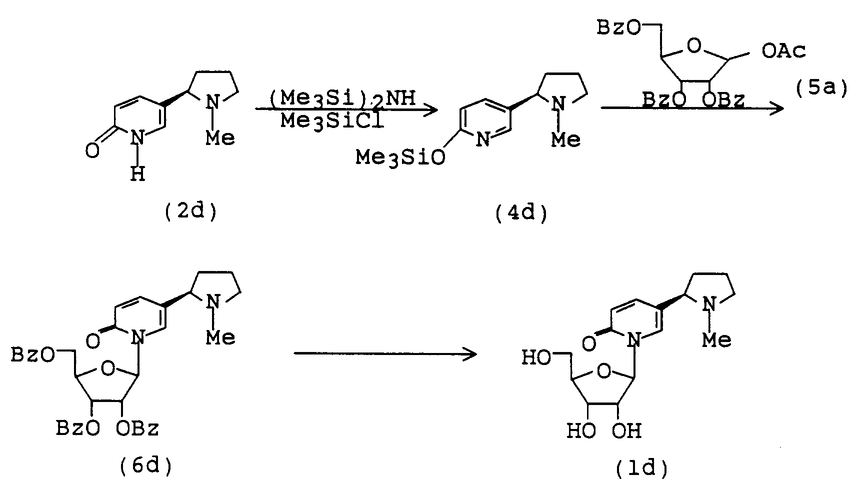
The compound (6d) is characterized in that it has an (R)-(+)-5-(1-methyl-2-pyrrolidinyl)-2-pyridone group as an aglycone and a 2,3,5-tri-O-benzoylribofuranosyl group as a glycosyl part, both of which are bound with β-N-glycosidic bond. The compound (6d) is an important intermediate to prepare the compound (1d) as well as a nicotine derivative having useful properties by itself.

### [E] Synthesis of (S)-(-)-3-(1-methyl-5-oxo-2-pyrrolidinyl)-2-pyridone-N-β-D-ribofuranoside (1e)

As shown in the reaction below, the compound (1e) can be obtained in the same manner as in the step [A] except that (S)-(-)-3-(1-methyl-5-oxo-2-pyrrolidinyl)-2(1H)-pyridone (2e) and 1-O-acetyl-2,3,5-tri-O-benzoyl-β-D-ribofuranose (5a) are used. The compounds (1c) and (6c) can be purified by means of column chromatography and the like. 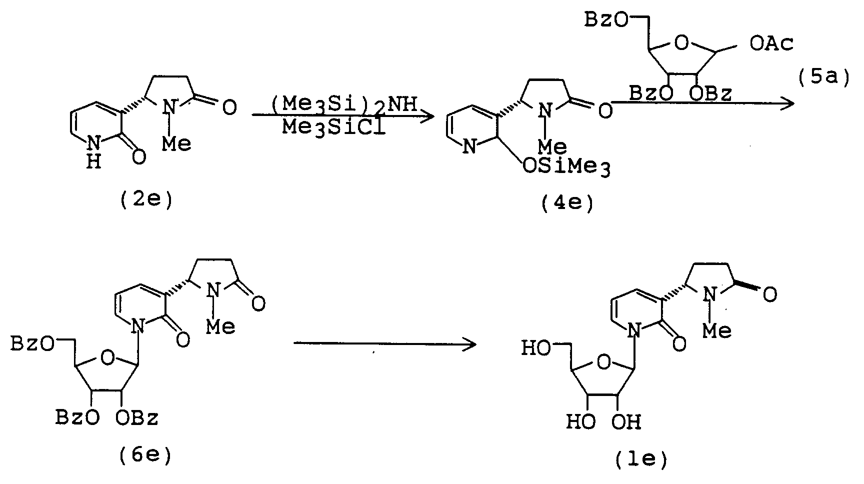
The compound (6e) is characterized in that it has an (S)-(-)-3-(1-methyl-5-oxo-2-pyrrolidinyl)-2-pyridone group as an aglycone and a 2,3,5-tri-O-benzoylribofuranosyl group as a glycosyl part, both of which are bound with β-N-glycosidic bond. The compound (6e) is an important intermediate to prepare the compound (1e) as well as a nicotine derivative having useful properties by itself.

### [F] Synthesis of (S)-(-)-5-(1-methyl-5-oxo-2-pyrrolidinyl)-2-pyridone-N-β-D-ribofuranoside (1f)

As shown in the reaction below, the compound (1f) can be obtained in the same manner as in the step [A] except that (S)-(-)-5-(1-methyl-5-oxo-2-pyrrolidinyl)-2(1H)-pyridone (2f) and 1-O-acetyl-2,3,5-tri-O-benzoyl-β-D-ribofuranose (5a) are used. The compounds (1f) and (6f) can be purified by means of column chromatography and the like. 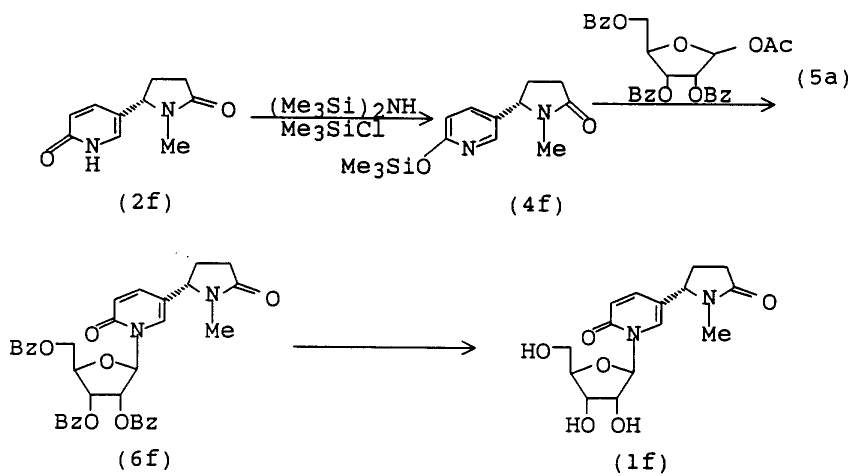
The compound (6f) is characterized in that it has an (S)-(-)-5-(1-methyl-5-oxo-2-pyrrolidinyl)-2-pyridone group as an aglycone and a 2,3,5-tri-O-benzoylribofura-nosyl group as a glycosyl part, both of which are bound with β-N-glycosidic bond. The compound (6f) is an important intermediate to prepare the compound (1f) as well as a nicotine derivative having useful properties by itself.

Hereinbelow, a novel nicotine derivative according to the second aspect of the present invention will be described.

The novel nicotine derivative of the present invention is a compound represented by the following general formula (2): 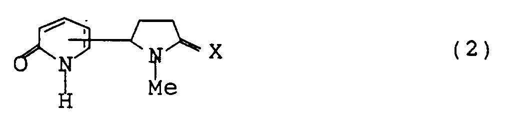
wherein X is the same as defined above.

In the present invention, the compound (2) is a useful compound by itself as well as an important starting material to produce the compound (1).

In the present invention, a nicotine derivative represented by the formula (2) is (1-methyl-2-pyrrolidinyl)-2(1H)-pyridone or (1-methyl-5-oxo-2-pyrrolidinyl)-2(1H)-pyridone is preferable since they are relatively easier to synthesize. More preferably, (S)-(1-methyl-2-pyrrolidinyl)-2(1H)-pyridone, (R)-(1-methyl-2-pyrrolidinyl)-2(1H)-pyridone, (S)-(1-methyl-5-oxo-2-pyrrolidinyl)-2(1H)-pyridone, or (R)-(1-methyl-5-oxo-2-pyrrolidinyl)-2(1H)-pyridone is used. In the present invention, particularly preferable novel nicotine derivative is an optically active (S)-(-)-3-(1-methyl-2-pyrrolidinyl)-2(1H)-pyridone (2a), (R)-(+)-3-(1-methyl-2-pyrrolidinyl)-2(1H)-pyridone (2b), (S)-(-)-5-(1-methyl-2-pyrrolidinyl)-2(1H)-pyridone (2c), (R)-(+)-5-(1-methyl-2- pyrrolidinyl)-2(1H)-pyridone (2d), (S)-(-)-3-(1-methyl-5-oxo-2-pyrrolidinyl)-2(1H)-pyridone (2e), or (S)-(-)-5-(1-methyl-5-oxo-2-pyrrolidinyl)-2(1H)-pyridone (2f). 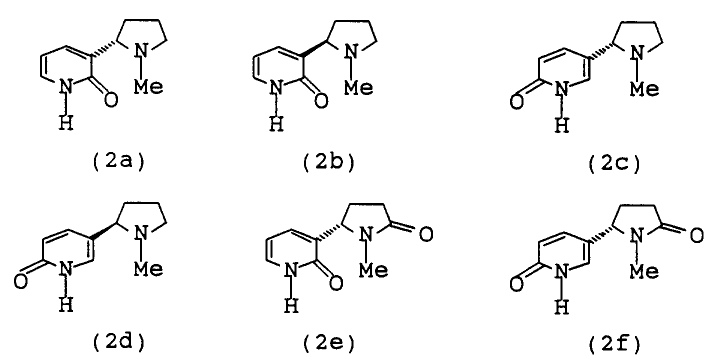
Hereinbelow, a method for producing the nicotine derivative (2) according to the second aspect of the present invention will be described with reference to methods for producing the preferable compounds (2a) to (2f) of the present invention, which should not be construed as limiting the scope of the present invention.

The pyridone derivatives (2) used as a starting material are classified into two, namely, (2a) to (2d) with a pyrrolidine ring whose 5-position is not oxidized, and (2e) and (2f) whose 5-position is oxidized as is seen in the above-formulas.

First, methods of compounds (2a), (2b), (2c) and (2d) whose pyrrolidine rings are not oxidized, will be described.

Compound (4a), (4b), (4c) and (4d) can be relatively easily obtained by the optical resolution of 3-(1-methyl-2-pyrrolidinyl)-2(1H)-pyridone (7) and 5-(1-methyl-2-pyrrolidinyl)-2(1H)-pyridone (8) which are prepared by a method shown in Chem. Ber., 57B, 1163 (1924) by means of an optically active column as shown in the following reactions. 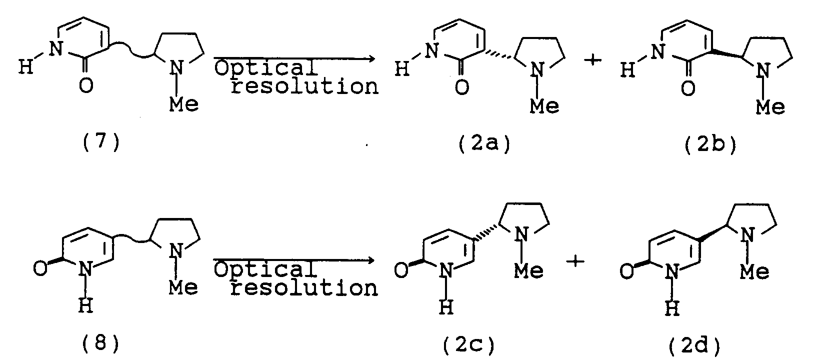
On the other hand, compounds (2e) and (2f) can be hydrolytically obtained by refluxing with heating nicotine-N-oxide (9) in acetic anhydride, which is manufactured by a known method (Shibagaki et al., Heterocycles, 23, 1681 (1985)) from nicotine as shown below, followed by hydrolyzing nicotine-N-oxide (9) with water or alcohol such as butanol, as shown below. The obtained product is isolated to purify by means of silica gel column chromatography, or the like. 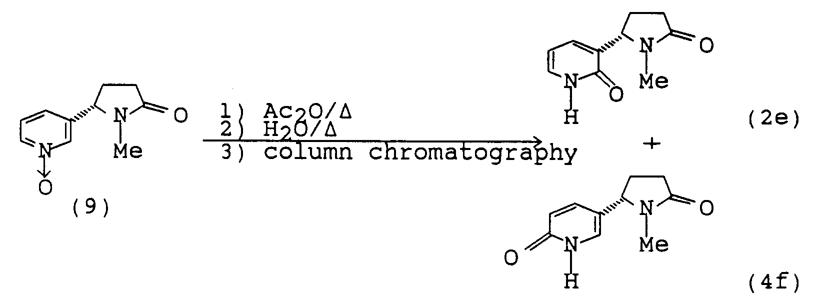
The compound (1) of the present invention and the intermediate thereof (6) are expected to have a variety of physiological activities, such as autonomic nervous regulatory activity, a circulatory-system regulatory activity such as a vasopressor activity, or a digestive organ regulatory activity such as gastrointestinal activation. The most practical usage is an improving agent of flavor and taste. The evaluation of the compound (1) of the present invention and the intermediate thereof (6) can be performed by the following 1) and 2) tests.
1) The effect on flavor, taste, and stimulation
   Ethanol solutions of the compound (1) and the intermediate thereof (6) are prepared. The ethanol solutions are spray-added to a shredded tobacco fillings. The tobacco fillings are rolled up and compared with a control, namely a non-sprayed tobacco filling in terms of flavor, taste, and stimulation by a discrimination method.
2) The effect on flavor in combination with taste
   An ethanol solution of the mixture of the compound (1) and the intermediate thereof (6) is prepared. The ethanol solution is spray-added to a shredded tobacco filling. The tobacco filling is rolled up and compared with a control, namely a non-sprayed tobacco filling in terms of flavor in combination with taste at the time of smoking.

The above-mentioned evaluation tests on flavor in combination with taste are performed using the preferable compounds (1a) to (1f), (6e) and (6f) of the present invention, it is found that they are excellent products as a flavor and taste improving agent.

### Best Mode of Carrying Out the Invention

Hereinbelow, the present invention will be described with reference to Examples, which should not be construed as limiting the scope of the present invention.

### Example 1

### 1. Synthesis of (S)-(-)-3-(1-methyl-2-pyrrolidinyl)-2(1H)-pyridone (2a) and (R)-(+)-3-(1-methyl-2-pyrrolidinyl)-2(1H)-pyridone (2b)

3-(1-methyl-2-pyrrolidinyl)-2(1H)-pyridone (7) were synthesized according to a method described in a known literature (Chem. Ber., 57B, 1163 (1924)).

(S)-(-)-3-(1-methyl-2-pyrrolidinyl)-2(1H)-pyridone (2a) and (R)-(+)-3-(1-methyl-2-pyrrolidinyl)-2 (1H)-pyridone (2b) were obtained by resolving 3-(1-methyl-2-pyrrolidinyl)-2(1H)-pyridone (7) using an optically active column.

### Separation condition

- Column:: CHIRALPAK AS (Daisel Chemical Industries, LTD)
- Solvent:: hexane/2-propanol = 9/1
- Retention time:: (S)-(-)-3-(1-methyl-2-pyrrolidinyl)-2(1H)-pyridone (2a); 19 minutes;
(R)-(+)-3-(1-methyl-2-pyrrolidinyl)-2(1H)-pyridone (2b); 14 minutes;

### Physical properties

(S)-(-)-3-(1-methyl-2-pyrrolidinyl)-2(1H)-pyridone (2a)
[α]_{D} - 197° (c 0.93, methanol)
Melting point: 95.0 - 96.0°C
(R)-(+)-3-(1-methyl-2-pyrrolidinyl)-2(1H)-pyridone (2b);
[α]_{D} + 207° (c 0.89, methanol)
Melting point: 93.0 - 93.5°C
The values of the physical properties of the compounds (2a) and (2b) are identical as shown in the following.
- IR:: ν(cm-¹); 3136, 2946, 2778, 1647, 1618, 1562, 1477, 1164, 1052, 766
- ¹H-NMR:: δ (CDCl₃; ppm from TMS)
1.53 - 1.57 (1H, m)
1.80 - 1.87 (2H, m)
2.26 (3H, s)
2.40 (2H, m)
3.23 (1H, m)
3.49 (1H, t, J = 8.3 Hz)
6.34 (1H, dd, J = 6.4, 7.0 Hz)
7.36 (1H, dd, J = 2.0, 6.4 Hz)
7.65 (1H, dd, J = 2.0, 7.0 Hz)
- ¹³C-NMR:: δ (CDCl₃; ppm from TMS);
22.8 (CH₂), 33.1 (CH₂), 40.8 (CH₃), 56.1 (CH₂), 64.1 (CH), 107.2 (CH), 132.7 (CH), 134.0 (C), 136.8 (CH), 165.0 (C)
- MS (%):: 178 (M⁺) (23), 177 (11), 163 (64), 149 (29), 122 (11), 120 (11), 108 (12), 84 (100), 57 (13)

### 2. Synthesis of (S)-(-)-5-(1-methyl-2-pyrrolidinyl)-2(1H)-pyridone (2c) and (R)-(+)-5-(1-methyl-2-pyrrolidinyl)-2(1H)-pyridone (2d)

5-(1-methyl-2-pyrrolidinyl)-2(1H)-pyridone (8) were synthesized according to the method described in a known literature (Chem. Ber., 57B, 1163 (1924).

(S)-(-)-5-(1-methyl-2-pyrrolidinyl)-2(1H)-pyridone (2c) and (R)-(+)-5-(1-methyl-2-pyrrolidinyl)-2(1H)-pyridone (2d) were obtained by resolving 5-(1-methyl-2-pyrrolidinyl)-2(1H)-pyridone (8) obtained according to the method of above-described literature, by use of an optically active column.

### Condition for separation

- Column:: CHIRALPAK AS (Daisel Chemical Industries, Ltd)
- Solvent:: Hexane/ethanol = 9/1
- Retention time:: (S)-(-)-5-(1-methyl-2-pyrrolidinyl)-2(1H)-pyridone(2c); 40 minutes
(R)-(+)-5-(1-methyl-2-pyrrolidinyl)-2(1H)-pyridone(2d); 50 minutes

### Physical properties

(S)-(-)-5-(1-methyl-2-pyrrolidinyl)-2(1H)-pyridone(2c)
[α]_{D} - 117° (c 0.87, methanol)
Melting point: 123.0 - 124.0°C
(R)-(+)-5-(1-methyl-2-pyrrolidinyl)-2(1H)-pyridone(2d)
[α]_{D} + 111° (c 0.71, methanol)
Melting point: 123.5 - 124.5°C
Physical properties of the compound (2c) was identical to those of the compound (2d) as follows:
- IR:: ν (cm⁻¹); 3126, 2948, 2782, 1661, 1624, 1549, 1462, 1307, 1218, 1044, 837, 600
- ¹H-NMR:: δ (CDCl₃; ppm from TMS);
1.70 - 1.85 (2H, m)
1.85 - 1.95 (1H, m)
2.00 - 2.10 (1H, s)
2.15 (3H, s)
2.25 (1H, q, J = 7.6 Hz)
2.84 (1H, t, J = 8.2 Hz)
3.19 (1H, t, J = 7.6 Hz)
6.60 (1H, d, J = 9.4 Hz)
7.30 (1H, d, J = 2.5 Hz)
7.57 (1H, dd, J = 2.5, 9.4 Hz)
- ¹³C - NMR:: δ (CDCl₃; ppm from TMS);
22.5 (CH₂), 33.9 (CH₂), 40.2 (CH₃), 56.7 (CH₂), 67.7 (CH), 120.5 (CH), 121.9 (C), 132.8 (CH), 141.5 (CH), 165.6 (C)
- MS (%):: 179 (55), 178 (M⁺) (100), 177 (85), 150 (24), 149 (90), 135 (22), 122 (14), 121 (16), 84 (61)

### 3. Synthesis of (S)-(-)-3-(1-methyl-5-oxo-2-pyrrolidinyl)-2(1H)-pyridone (2e) and (S)-(-)-5-(1-methyl-5-oxo-2-pyrrolidinyl)-2(1H)-pyridone (2f)

1.7 g of nicotine-N-oxide (9) prepared according to a known method (Shibagaki et al., Heterocycles, 23, 1681 (1985)) was dissolved in 10 mℓ of acetic anhydride, and refluxed for 5 hours, followed by removing a solvent under reduced pressure. To the obtained residue, 20 mℓ of water was added and the mixture was refluxed for one hour and removed solvent under reduced pressure. The residue was purified by silica gel column chromatography (developing solvent: chloroform:methanol = 10 : 1), thereby obtaining the compound (2e) (1.2 g; 70% yield) and the compound (2f)(0.4 g: 25% yield).

### Physical properties

(S)-(-)-3-(1-methyl-5-oxo-2-pyrolidinyl)-2(1H)-pyridone(2e)
[α]_{D} - 144° (c 1.41, methanol)
- IR:: ν (cm⁻¹); 1649, 1611, 1562, 775
- ¹H - NMR:: δ (CDCl₃; ppm from TMS);
1.80 (1H, m)
2.40 (3H, m)
2.70 (3H, s)
4.77 (1H, dd, J = 3.4, 8.4 Hz)
6.29 (1H, dd, J = 6.4, 6.9 Hz)
7.15 (1H, dd, J = 1.9, 6.9 Hz)
7.35 (1H, dd, J = 1.9, 6.4 Hz)
- ¹³C - NMR:: δ (CDCl₃; ppm from TMS);
25.9 (CH₂), 28.2 (CH₃), 29.4 (CH₂), 58.8 (CH), 106.5 (CH), 130.6 (C), 133.7 (CH), 136.3 (CH), 163.5 (C), 176.0 (C)
- MS (%):: 193 (M⁺¹) (14), 192, (85), 177 (72), 164 (29), 163 (82), 149 (100), 135 (23), 134 (21), 121 (51), 120 (48), 108 (30), 98 (80), 78 (19)

| High resolution mass spectrum | |
|---|---|
| Calc · C₁₀H₁₃N₂O₂ | = 193.0977 |
| Obs. | 193.0987 |

(S)-(-)-5-(1-methyl-5-oxo-2-pyrrolidinyl)-2(1H)-pyridone (2f)
[α]_{D} + 22° (c 0.44, methanol)
- IR:: ν (cm⁻¹); 1657, 1618
- ¹H - NMR:: δ (CDCl₃; ppm from TMS);
1.90 (1H, m)
2.45 (1H, m)
2.50 (2H, m)
2.66 (3H, s)
4.13 (1H, dd, J = 6.2, 7.6 Hz)
6.67 (1H, d, J = 9.4 Hz)
7.28 (1H, d, J = 2.6 Hz)
7.36 (1H, dd, J = 2.6, 9.4 Hz)
- ¹³C - NMR:: dd (CDCl₃; ppm from TMS);
27.3 (CH₂), 28.0 (CH₃), 30.1 (CH₂), 61.2 (CH), 119.6 (C), 121.6 (CH), 132.9 (CH), 139.8 (CH), 165.0 (C), 175.4 (C)
- MS (%):: 192 (M⁺) (44), 191 (34), 163 (22), 137 (24), 136 (22), 135 (77), 98 (100), 81 (29), 80 (50), 79 (36), 77 (31), 69 (39), 68 (35), 67 (33), 66 (32), 65 (30)

| High resolution mass spectrum | |
|---|---|
| Calc · C₁₀H₁₃N₂O₂ | = 193.0977 |
| Obs. | 193.0998 |

### Example 2

### (S)-(-)-3-(1-methyl-2-pyrrolidinyl)-2-pyridone-N-β-D-ribofuranoside (1a)

### 1. Preparation of (S)-(-)-3-(1-methyl-2-pyrrolidinyl)-2-pyridone-2,3,5-tri-O-benzoyl-N-β-D-ribofuranoside (6a)

2.0 g of (S)-(-)-3-(1-methyl-2-pyrrolidinyl)-2(1H)-pyridone (2a) was added to 10 mℓ of hexamethyldisilazane. To this mixture, 1 mℓ of trimethylsilyl chloride was added, and then the mixture was refluxed for one hour. After completion of the reaction, the reaction mixture was concentrated under reduced pressure, thereby obtaining a trimethylsilyl derivative of the compound (2a) in oil state. This derivative was dissolved in 20 mℓ of 1,2-dichloroethane, to which 6.0 g of commercially available 2,3,5-tri-O-benzoyl-1-O-acetylribofuranose (5a) was added. Further, 12 mℓ of 1 M tin tetrachloride was added thereto, and the mixture was reacted for 5 hours. After 40 mℓ of dichloromethane was added to the reaction mixture, the reaction mixture was neutralized with 1 N sodium hydroxide. An organic layer was separated and washed with water. The organic layer was dried over anhydrous magnesium sulfate and concentrated under reduced pressure. The residue was purified by means of silica gel column chromatography using ethyl acetate as a developing solvent, thereby obtaining 6.1 g of (S)-(-)-3-(1-methyl-2-pyrrolidinyl)-2-pyridone-2,3,5-tri-O-benzoyl-N-β-D-ribofuranoside (6a) in white solid form.

### Physical properties

[α]_{D} - 10.7° (c 0.72, methanol)
- IR:: ν (cm⁻¹); 1734, 1655, 1602, 1560, 1454, 1267, 1096, 710
- ¹H - NMR:: δ (CDCl₃; ppm from TMS);
1.50 (1H, m)
1.80 (2H, m)
2.24 (3H, s)
2.32 (2H, m)
3.20 (1H, m)
3.47 (1H, m)
4.68 (1H, dd, J = 3.9, 12.3 Hz)
4.78 (1H, m)
4.89 (1H, dd, J = 2.8, 12.3 Hz)
5.84 (1H, dd, J = 3.8, 5.7 Hz)
5.95 (1H, dd, J = 6.0, 5.7 Hz)
6.14 (1H, t, J = 6.9 Hz)
6.59 (1H, d, J = 3.8 Hz)
7.34 (2H, t, J = 7.8 Hz)
7.39 (2H, t, J = 7.7 Hz)
7.55 (7H, m)
7.92 (2H, dd, J = 1.3, 7.1 Hz)
7.98 (2H, dd, J = 1.4, 7.1 Hz)
8.11 (2H, dd, J = 1.4, 7.2 Hz)
- ¹³C - NMR:: δ (CDCl₃; ppm from TMS);
22.7 (CH₂), 32.7 (CH₂), 40.7 (CH₃), 56.8 (CH₂), 63.4 (CH₂), 64.0 (CH), 70.7 (CH), 75.0 (CH), 79.9 (CH), 89.1 (CH), 106.4 (CH), 128.4 (CH), 128.6 (CH), 128.8 (C), 129.3 (C), 129.7 (CH), 129.8 (CH), 130.3 (CH), 133.4 (CH), 133.5 (CH), 134.7 (C), 135.1 (CH), 161.9 (C), 165.2 (C), 166.0 (C)
- MS (%):: 622 (M⁺) (2), 607 (6), 501 (18), 445 (12), 201 (16), 178 (12), 177 (95), 105 (100), 84 (12), 77 (31)
In the above physical properties, since absorption for a carbonyl group of the compound (6a) in IR spectrum is observed in the vicinity of 1655 cm⁻¹ and a characteristic signal of β-N-furanoside (δ 6.59 (1H, d, J = 3.8Hz)) is observed in ¹H-NMR spectrum, the structure of the compound (6a) can be confirmed.

### 2. Synthesis of (S)-(-)-3-(1-methyl-2-pyrrolidinyl)-2-pyridone-N-β-D-ribofuranoside (1a)

6.1 g of (S)-(-)-3-(1-methyl-2-pyrrolidinyl)-2-pyridone-2,3,5-tri-O-benzoyl-N-β-D-ribofuranoside (6a) was dissolved in 20 mℓ of methanol. To this, 10 mℓ of ammonia-saturated methanol was added and stirred at room temperature for 2 hours. The reaction mixture was concentrated under reduced pressure and the residue was purified by a silica gel column chromatography (methanol : chloroform = 1 : 3), thereby obtaining 3.0 g of (S)-(-)-3-(1-methyl-2-pyrrolidinyl)-2-pyridone-N-β-D-ribofuranoside (1a) in white solid form.

### Physical properties

[α]_{D} - 27.5° (c 1.7, methanol)
- UV:: λₘₐₓ 204 nm (ε = 6,300), 226 nm (ε = 3,400), 308 nm (ε = 3,200)
- IR:: ν (cm⁻¹) 3340, 2940, 1649, 1582, 1555, 1456, 1386, 1265, 1197, 1108, 1054, 768, 717
- ¹H-NMR:: δ (CDCl₃; ppm from TMS);
1.47 (1H, m)
1.77 (2H, m)
2.14 (3H, s)
2.26 (1H, m)
2.27 (1H, q, J = 9.2 Hz)
3.12 (1H, m)
3.40 (1H, t, J = 8.4 Hz)
3.70 (1H, dd, J = 3.1, 12.4 Hz)
3.86 (1H, dd, J = 2.4, 12.4 Hz)
4.05 (3H, m)
6.04 (1H, d, J = 3.0 Hz)
6.33 (1H, t, J = 7.0 Hz)
7.49 (1H, dd, J = 1.7, 6.9 Hz)
7.96 (1H, dd, J = 1.7, 7.0 Hz)
- ¹³C - NMR:: δ (CDCl₃; ppm from TMS);
23.1 (CH₂), 33.2 (CH₂), 40.9 (CH₃), 57.7 (CH₂), 61.5 (CH₂), 65.6 (CH), 70.2 (CH), 76.8 (CH), 85.6 (CH), 91.9 (CH), 107.7 (CH), 133.0 (CH), 133.1 (C), 137.3 (CH), 163.6 (C)
- MS (%):: 311 (M⁺¹) (1), 310 (4), 295 (16), 219 (19), 179 (11), 178 (16), 177 (100), 163 (68), 149 (25), 148 (19), 122 (26), 84, (35), 57 (26)
In the above physical properties, since absorption for a carbonyl group of the compound (1a) in IR spectrum is observed in the vicinity of 1649 cm⁻¹, the structure of the compound (1a) can be confirmed.

### Example 3

### (R)-(+)-3-(1-methyl-2-pyrrolidinyl)-2-pyridone-N-β-D-ribofuranoside (1b)

### 1. Preparation of (R)-(+)-3-(1-methyl-2-pyrrolidinyl)-2-pyridone-2,3,5-tri-O-benzoyl-N-β-D-ribofuranoside (6b)

2.0 g of (R)-(+)-3-(1-methyl-2-pyrrolidinyl)-2(1H)-pyridone (2b) was converted to the trimethylsilyl derivative of compound (2b) in the same manner as in Example 2, step 1. To this derivative, 6.0 g of 2,3,5-tri-O-benzoyl-1-O-acetylribofuranose (5a) and 12 mℓ of 1 M tin tetrachloride were added. The reaction and purification were carried out in the same manner as in Example 2, step 1, thereby obtaining 6.0 g of (R)-(+)-3-(1-methyl-2-pyrrolidinyl)-2-pyridone-2,3,5-tri-O-benzoyl-N-β-D-ribofuranoside (6b).

### Physical properties

[α]_{D} + 99° (c 0.7 methanol)
- IR:: ν (cm⁻¹); 1734, 1655, 1603, 1560, 1452, 1267, 1096, 710
- ¹H - NMR:: δ (CDCl₃; ppm from TMS);
1.47 (1H, m)
1.80 (2H, m)
2.25 (3H, s)
2.35 (2H, m)
3.19 (1H, m)
3.46 (1H, t, J = 8.0 Hz)
4.68 (1H, dd, J = 3.8, 12.4 Hz)
4.79 (1H, m)
4.89 (1H, dd, J = 2.8, 12.4 Hz)
5.87 (1H, m)
5.93 (1H, t, J = 6.0 Hz)
6.14 (1H, t, J = 6.9 Hz)
6.60 (1H, d, J = 3.5 Hz)
7.33 (2H, t, J = 7.5 Hz)
7.39 (2H, t, J = 7.5 Hz)
7.25 (6H, m)
7.61 (1H, t, J = 7.1 Hz)
7.91 (2H, dd, J = 1.7, 7.5 Hz)
7.99 (2H, dd, J = 1.7, 7.5 Hz)
8.11 (2H, dd, J = 1.7, 7.5 Hz)
- ¹³C - NMR:: δ (CDCl₃; ppm from TMS);
22.7 (CH₂), 32.5 (CH₂), 40.8 (CH₃), 56.9 (CH₂), 63.3 (CH₂), 64.0 (CH), 70.5 (CH), 74.9 (CH), 79.7 (CH), 89.1 (CH), 106.4 (CH), 128.4 (CH), 128.5 (CH), 128.8 (C), 129.3 (C), 129.7 (CH), 129.8 (CH), 130.1 (CH), 133.4 (CH), 133.5 (CH), 134.8 (CH), 135.0 (C), 161.9 (C), 165.1 (C), 165.2 (C), 166.0 (C)
- MS (%):: 622 (M⁺) (2), 607 (7), 501 (19), 445 (13), 201 (16), 178 (13), 177 (100), 105 (93), 84 (12), 77 (27)
In the above physical properties, since absorption for a carbonyl group of the compound (6b) in IR spectrum is observed in the vicinity of 1655 cm⁻¹, the structure of the compound (6b) can be confirmed.

### 2. Synthesis of (R)-(+)-3-(1-methyl-2-pyrrolidinyl)-2-pyridone-N-β-D-ribofuranoside (1b)

6.1 g of (R)-(+)-3-(1-methyl-2-pyrrolidinyl)-2-pyridone-2,3,5-tri-O-benzoyl-N-β-D-ribofuranoside (6b) was dissolved in 20 mℓ of methanol. This was treated with ammonia-saturated methanol and purified in the same manner as in Example 2, step 2, thereby obtaining 2.9 g of (R)-(+)-3-(1-methyl-2-pyrrolidinyl)-2-pyridone-N-β-D-ribofuranoside (1b) in white solid form.

### Physical properties

[α]_{D} + 209° (c 1.7, methanol)
- UV:: λₘₐₓ 205 nm (ε = 9,300), 228 nm (ε = 4,700), 308 nm (ε = 6,200)
- IR:: ν (cm⁻¹) 3340, 2934, 1649, 1580, 1557, 1458, 1386, 1263, 1197, 1108, 1054, 766
- ¹H - NMR:: δ (CDCl₃; ppm from TMS);
1.45 (1H, m)
1.75 (2H, m)
2.17 (3H, s)
2.25 (1H, m)
2.31 (1H, q, J = 9.0 Hz)
3.14 (1H, m)
3.45 (1H, t, J = 8.5 Hz)
3.71 (1H, dd, J = 3.1, 12.4 Hz)
3.86 (1H, dd, J = 2.4, 12.4 Hz)
4.05 (3H, m)
6.05 (1H, d, J = 2.9 Hz)
6.33 (1H, t, J = 7.0 Hz)
7.49 (1H, dd, J = 1.7, 6.9 Hz)
7.96 (1H, dd, J = 1.7, 7.0 Hz)
- ¹³C - NMR:: δ (CDCl₃; ppm from TMS);
23.0 (CH₂), 33.0 (CH₂), 40.9 (CH₃), 57.7 (CH₂), 61.5 (CH₂), 65.7 (CH), 70.2 (CH), 76.7 (CH), 85.6 (CH), 91.8 (CH), 107.7 (CH), 132.8 (C), 133.0 (CH), 137.4 (CH), 163.5 (C)
- MS (%):: 311 (M⁺¹) (1), 310 (5), 295 (18), 219 (21), 179 (12), 178 (14), 177 (100), 163 (69), 149 (18), 148 (19), 122 (23), 84 (28), 57 (22)
In the above physical properties, since absorption for a carbonyl group of the compound (1b) in IR spectrum is observed in the vicinity of 1649 cm⁻¹, the structure of the compound (1b) can be confirmed.

### Example 4

### (S)-(-)-5-(1-methyl-2-pyrrolidinyl)-2-pyridone-N-β-D-ribofuranoside (1c)

### 1. Preparation of (S)-(-)-5-(1-methyl-2-pyrrolidinyl)-2-pyridone-2,3,5-tri-O-benzoyl-N-β-D-ribofuranoside (6c)

2.0 g of (S)-(-)-5-(1-methyl-2-pyrrolidinyl)-2(1H)-pyridone (2c) was converted to the trimethylsilyl derivative of the compound (2c) in the same manner as in Example 2, step 1. To this derivative, 6.0 g of 2,3,5-tri-O-benzoyl-1-O-acetylribofuranose (5a) and 12 mℓ of 1 M tin tetrachloride were added. The reaction and purification were carried out in the same manner as in Example 2, step 1, thereby obtaining 6.1 g of (S)-(-)-5-(1-methyl-2-pyrrolidinyl)-2-pyridone-2,3,5-tri-O-benzoyl-N-β-D-ribofuranoside (6c) in white solid form.

### 2. Synthesis of (S)-(-)-5-(1-methyl-2-pyrrolidinyl)-2-pyridone-N-β-D-ribofuranoside (1c)

6.1 g of (S)-(-)-5-(1-methyl-2-pyrrolidinyl)-2-pyridone-2,3,5-tri-O-benzoyl-N-β-D-ribofuranoside (6c) was dissolved in 20 mℓ of methanol. This was treated with ammonia-saturated methanol and purified in the same manner as in Example 2, step 2, thereby obtaining 3.0 g of (S)-(-)-5-(1-methyl-2-pyrrolidinyl)-2-pyridone-Nβ-D-ribofuranoside (1c) in white solid form.

### Physical properties

[α]_{D} + 1.4° (c 0.79, methanol)
- UV:: λₘₐₓ 206 nm (ε= 8,300), 230 nm (ε = 7,700), 308 nm (ε = 4,300)
- IR:: ν (cm⁻¹), 3292, 2950, 1663, 1578, 1543, 1108, 1060, 837
- ¹H - NMR:: δ (CDCl₃; ppm from TMS);
1.60 (1H, m)
1.70 - 1.90 (2H, m)
2.10 (3H, s)
2.15 (1H, m)
2.22 (1H, q, J = 7.8 Hz)
2.86 (1H, t, J = 7.8 Hz)
3.12 (1H, t, J = 7.6 Hz)
3.65 (1H, m)
3.83 (1H, m)
4.0 (3H, m)
6.01 (1H, d, J = 3.0 Hz)
6.40 (1H, d, J = 9.4 Hz)
7.41 (1h, dd, J = 2.4, 9.4 Hz)
7.92 (1H, d, J = 2.4 Hz)
- ¹³C - NMR:: δ (CDCl₃; ppm from TMS);
21.2 (CH₂), 32.1 (CH₂), 38.9 (CH₃), 55.8 (CH₂), 59.7 (CH₂), 67.4 (CH), 68.3 (CH), 75.0 (CH), 83.9 (CH), 90.4 (CH), 119.2 (CH), 119.9 (C), 131.6 (CH), 139.8 (CH), 162.4 (C)
- MS (%):: 310 (M⁺) (18), 309 (5), 280 (5), 221, (12), 179 (48), 178 (58), 177 (91), 150 (35), 149 (69), 136 (20), 135 (28), 134 (11), 84 (100)
In the above physical properties, since absorption for a carbonyl group of the compound (1c) in IR spectrum is observed in the vicinity of 1663 cm⁻¹ and a characteristic signal of β-N-furanoside (δ 6.01 (1H, d, J = 3.0Hz)) is observed in ¹H-NMR spectrum, the structure of the compound (1c) can be confirmed.

### Example 5

### (R)-(+)-3-(1-methyl-2-pyrrolidinyl)-2-pyridone-N-β-D-ribofuranoside (1d)

### 1. Preparation of (R)-(+)-5-(1-methyl-2-pyrrolidinyl)-2-pyridone-2,3,5-tri-O-benzoyl-N-β-D-ribofuranoside (6d)

2.0 g of (R)-(+)-5-(1-methyl-2-pyrrolidinyl)-2(1H)-pyridone (2d) was converted to the trimethylsilyl derivative of compound (2d) in the same manner as in Example 2, step 1. To this derivative, 6.0 g of 2,3,5-tri-O-benzoyl-1-O-acetylribofuranose (5a) and 12 mℓ of 1 M tin tetrachloride were added. The reaction and purification were performed in the same manner as in Example 2, step 1, thereby obtaining 6.0 g of (R)-(+)-5-(1-methyl-2-pyrrolidinyl)-2-pyridone-2,3,5-tri-O-benzoyl-N-β-D-ribofuranoside (6d) in white solid form.

### 2. Synthesis of (R)-(+)-3-(1-methyl-2-pyrrolidinyl)-2-pyridone-N-β-D-ribofuranoside (1d)

6.0 g of (R)-(+)-5-(1-methyl-2-pyrrolidinyl)-2-pyridone-2,3,5-tri-O-benzoyl-N-β-D-ribofuranoside (6d) was dissolved in 20 mℓ of methanol. This was treated with ammonia-saturated methanol and purified in the same manner as in Example 2, step 2, thereby obtaining 2.9 g of (R)-(+)-5-(1-methyl-2-pyrrolidinyl)-2-pyridone-N-β-D-ribofuranoside (1d) in white solid form.

### Physical properties

[α]_{D} + 79.6° (c 0.89, MeOH)
- UV:: λₘₐₓ 206 nm (ε = 6,800), 230 nm (ε = 5,800), 308 nm (ε = 3,300)
- IR:: (cm⁻¹) 3342, 2950, 1663, 1582, 1543, 1108, 1060, 839
- ¹H - NMR:: δ (CDCl₃; ppm from TMS);
1.55 - 1.80 (3H, m)
2.03 (1H, m)
2.08 (3H, s)
2.20 (1H, q, J = 7.7 Hz)
2.85 (1H, t, J = 7.7 Hz)
3.11 (1H, t, J = 7.6 Hz)
3.63 (1H, m)
3.79 (1H, m)
4.0 (3H, m)
5.98 (1H, d, J = 3.0 Hz)
6.37 (1H, d, J = 9.3 Hz)
7.40 (1H, dd, J = 2.3, 9.3 Hz)
7.96 (1H, d, J = 2.3 Hz)
- ¹³C - NMR:: δ (CDCl₃; ppm from TMS);
21.4 (CH₂), 32.2 (CH₂), 39.1 (CH₃), 56.1 (CH₂), 59.7 (CH₂), 67.9 (CH), 68.1 (CH), 75.1 (CH), 84.1 (CH), 91.0 (CH), 119.3 (CH), 119.8 (C), 132.1 (CH), 140.4 (CH), 162.6 (C)
- MS (%):: 310 (M⁺) (10), 309 (5), 280 (13), 221 (15), 179 (46), 178 (59), 177 (94), 150 (36), 149 (71), 136 (20), 135 (28), 84 (100)
In the above physical properties, since absorption for a carbonyl group of the compound (1d) in IR spectrum is observed in the vicinity of 1663 cm⁻¹ and a characteristic signal of β-N-furanoside (δ 5.98 (1H, d, J = 3.0Hz)) is observed in ¹H-NMR spectrum, the structure of the compound (1d) can be confirmed.

### Example 6

### (S)-(-)-3-(1-methyl-5-oxo-2-pyrrolidinyl)-2-pyridone-N-β-D-ribofuranoside (1e)

### 1. Preparation of (S)-(-)-3-(1-methyl-5-oxo-2-pyrrolidinyl)-2-pyridone-2,3,5-tri-O-benzoyl-N-β-D-ribofuranoside (6e)

1.0 g of (S)-(-)-3-(1-methyl-5-oxo-2-pyrrolidinyl)-2(1H)-pyridone (2e) was added to 10 mℓ of hexamethyldisilazane. To this mixture, 0.5 mℓ of trimethylsilyl chloride was added. After refluxed for one hour, the reaction mixture was concentrated under reduced pressure, thereby obtaining a trimethylsilyl derivative of the compound (2e) in oil state. The obtained derivative was dissolved in 20 mℓ of 1,2-dichloroethane. To this solution, 3.0 g of commercially available 2,3,5-tri-O-benzoyl-1-O-acetylribofuranose (5a) and 6 mℓ of 1 M tin tetrachloride were successively added. After the mixture was reacted for 5 hours, 40 mℓ of dichloromethane was added to the reaction mixture. The mixture was then neutralized with a 1 N sodium hydroxide solution. An organic layer was separated and washed with water, and dried over anhydrous magnesium sulfate. After removal of a solvent under reduced pressure, the residue was purified by means of silica gel column chromatography (developing solvent: chloroform : methanol = 10 : 1) thereby obtaining 2.5 g (75 % yield) of (S)-(-)-3-(1-methyl-5-oxo-2-pyrrolidinyl)-2-pyridone-2,3,5-O-benzoyl-N-β-D-ribofuranoside (6e) in white solid form.

### Physical properties

[α]_{D} - 8.0° (c 0.74, methanol)
- UV:: λₘₐₓ 203 nm (ε = 60,000), 218 nm (ε = 46,000), 230 nm (40,000)
- IR:: ν (cm⁻¹); 1729, 1661, 1603, 1560, 1270, 712
- ¹H - NMR:: δ (CDCl₃; ppm from TMS);
1.79 (1H, m)
2.40 (3H, m)
2.75 (3H, s)
4.17 (1H, dd, J = 3.8, 12.3 Hz)
4.81 (2H, m)
4.91 (1H, dd, J = 2.8, 12.3 Hz)
5.87 (1H, dd, J = 4.0, 5.8 Hz)
5.98 (1H, t, J = 5.8 Hz)
6.14 (1H, t, J = 6.9 Hz)
6.58 (1H, d, J = 4.0 Hz)
7.06 (1H, dd, J = 1.6, 6.9 Hz)
7.36 (2H, t, J = 7.8 Hz)
7.39 (2H, t, J = 7.8 Hz)
7.47 (2H, t, J = 7.8 Hz)
7.60 (4H, m)
7.95 (2H, dd, J = 1.2, 8.3 Hz)
7.98 (2H, dd, J = 1.1, 8.3 Hz)
8.12 (2H, dd, J = 1.2, 8.4 Hz)
- ¹³C - NMR:: δ (CDCl₃; ppm from TMS);
25.7 (CH₂), 28.5 (CH₃), 29.4 (CH₂), 59.0 (CH), 63.3 (CH₂), 70.7 (CH), 75.0 (CH), 80.1 (CH), 89.1 (CH), 105.9 (CH), 128.5 (CH), 128.6 (CH), 129.3 (CH), 129.7 (CH), 129.8 (CH), 131.8 (CH), 133.6 (C), 133.7 (C), 135.6 (CH), 160.9 (C), 165.3 (C), 166.0 (C), 166.1 (C), 176.0 (C)
- MS (%):: 636 (M⁺) (2), 635 (2), 446 (6), 445 (21), 270 (6), 201 (23), 191 (34), 122 (15), 105 (100), 77 (32)

### 2. Synthesis of (S)-(-)-3-(1-methyl-5-oxo-2-pyrrolidinyl)-2-pyridone-N-β-D-ribofuranoside (1e)

2.5 g of (S)-(-)-3-(1-methyl-5-oxo-2-pyrrolidinyl)-2-pyridone-2,3,5-O-benzoyl-N-β-D-ribofuranoside (6e) was dissolved in 20 mℓ of methanol. To this, 10 mℓ of ammonia-saturated methanol was added and stirred at room temperature for 2 hours. The reaction mixture was concentrated under reduced pressure, thereby obtaining 1.1 g (90% yield) of (S)-(-)-3-(1-methyl-5-oxo-2-pyrrolidinyl)-2-pyridone-N-β-D-ribofuranoside (1e) in white solid form.

### Physical properties

[α]_{D} + 1.2° (c 0.58, methanol)
- UV:: λₘₐₓ 205 nm (ε = 78,000), 217 nm (ε = 55,000)
- IR:: ν (cm⁻¹); 3356, 1649, 1560, 1390, 1110, 1056
- ¹H - NMR:: δ (CDCl₃; ppm from TMS);
1.91 (1H, m)
2.50 (3H, m)
2.74 (3H, s)
3.83 (1H, dd, J = 2.8, 12.4 Hz)
3.99 (1H, dd, J = 2.0, 12.4 Hz)
4.15 (2H, m)
4.21 (1H, dd, J = 5.1, 6.4 Hz)
4.82 (1H, m)
6.12 (1H, d, J = 2.7 Hz)
6.43 (1H, t, J = 6.9 Hz)
7.25 (1H, dd, J = 1.6, 6.9 Hz)
8.16 (1H, dd, J = 1.6, 7.0 Hz)
- ¹³C - NMR:: δ (CDCl₃; ppm from TMS);
24.5 (CH₂), 27.4 (CH₃), 29.1 (CH₂), 59.8 (CH₂), 59.8 (CH), 66.4 (CH), 75.2 (CH), 84.0 (CH), 90.4 (CH), 105.7 (CH), 129.1 (C), 132.6 (CH), 135.7 (CH), 160.9 (C), 176.5 (C)
- MS (%):: 324 (M⁺) (7), 233 (12), 193 (12), 192 (34), 191 (100), 177 (25), 164 (12), 163 (50), 149 (21), 134 (24), 121 (14), 120 (14), 98 (35)

### Example 7

### (S)-(-)-5-(1-methyl-5-oxo-2-pyrrolidinyl)-2-pyridone-N-β-D-ribofuranoside (1f)

### 1. Preparation of (S)-(-)-5-(1-methyl-5-oxo-2-pyrrolidinyl)-2-pyridone-2,3,5-tri-O-benzoyl-N-β-D-ribofuranoside (6f)

A trimethylsilyl derivative of (S)-(-)-5-(1-methyl-5-oxo-2-pyrrolidinyl)-2(1H)-pyridone (2f) obtained in Example 1, step 3 was obtained in oil state from 1.0 g of the compound (2f) through the same reaction procedure as Example 6, step 1. The obtained derivative was dissolved in 20 mℓ of 1,2-dichloroethane, to which 3.0 g of commercially available 2,3,5-tri-O-benzoyl-1-O-acetylribofuranose (5a) was added. Thereafter, a same reaction and work-up were carried out in the same manner as in Example 6, step 1, thereby obtaining 2.5 g (75% yield) of (S)-(-)-5-(1-methyl-5-oxo-2-pyrrolidinyl)-2-pyridone-2,3,5-O-benzoyl-N-β-D-ribofuranoside (6f).

### Physical properties

[α]_{D} + 8.1° (c 0.95, methanol)
- UV:: λₘₐₓ 203 nm (ε = 49,000), 217 nm (ε = 40,000), 231 nm (27,000)
- IR:: ν (cm⁻¹), 1727, 1678, 1618, 1454, 1270, 1096, 712
- ¹H - NMR:: δ (CDCl₃; ppm from TMS);
1.70 (1H, m)
2.20 (1H, m)
2.34 (1H, q, J = 9.1 Hz)
2.46 (1H, m)
2.46 (3H, s)
3.60 (1H, t, J = 7.3 Hz)
4.64 (1H, dd, J = 3.6, 12.3 Hz)
4.78 (1H, ddd, J = 2.7, 3.6, 4.6 Hz)
5.03 (1H, dd, J = 2.7, 12.3 Hz)
5.82 (1H, dd, J = 5.5, 6.2 Hz)
5.98 (1H, dd, J = 4.6, 6.2 Hz)
6.68 (1H, d, J = 5.5 Hz)
7.11 (1H, dd, J = 2.5, 9.5 Hz)
7.31 (1H, d, J = 2.5 Hz)
7.38 (2H, m)
7.40 (2H, m)
7.50 (2H, m)
7.56 (3H, m)
7.97 (4H, m)
8.17 (2H, d, J = 7.0 Hz)
- ¹³C - MNR:: δ (CDCl₃; ppm from TMS);
27.0 (CH₂), 27.8 (CH₃), 30.2 (CH₂), 61.1 (CH), 63.8 (CH₂), 71.5 (CH), 74.7 (CH), 80.7 (CH), 87.7 (CH), 118.9 (C), 122.7 (CH), 128.6 (CH), 128.9 (CH), 129.5 (C), 129.7 (CH), 129.9 (CH), 130.0 (CH), 130.7 (CH), 133.8 (CH), 137.4 (CH), 161.7 (C), 165.3 (C), 166.0 (C), 168.0 (C), 175.1 (C)
- MS (%):: 446 (3), 445 (10), 201 (11), 122 (30), 106 (10), 105 (100), 77 (48)

### 2. Synthesis of (S)-(-)-5-(1-methyl-5-oxo-2-pyrrolidinyl)-2-pyridone-N-β-D-ribofuranoside (1f)

(S)-(-)-5-(1-methyl-5-oxo-2-pyrrolidinyl)-2-pyridone-2,3,5-O-benzoyl-N-β-D-ribofuranoside (6f) obtained in Example 7, step 1, was treated in the same manner as in Example 6, step 2. 2.5 g of the compound (6f) was dissolved in 20 mℓ of methanol, to which 10 mℓ of ammonia-saturated methanol was added and stirred at room temperature for 2 hours. The reaction mixture was concentrated under reduced pressure, thereby obtaining 1.1 g (90% yield) of (S)-(-)-5-(1-methyl-5-oxo-2-pyrrolidinyl)-2-pyridone-N-β-D-ribofuranoside (1f) in white solid form.

### Physical properties

[α]_{D} + 66° (c 0.61, methanol)
- UV:: λₘₐₓ 206 nm (ε = 27,000), 225 nm (ε = 13,000), 315 nm (ε = 4,000)
- IR:: ν (cm⁻¹); 3350, 1660, 1585, 1106
- ¹H - NMR:: δ (CDCl₃; ppm from TMS);
1.92 (1H, m)
2.49 (2H, m)
2.58 (1H, m)
2.64 (3H, s)
3.83 (1H, dd, J = 2.4, 12.4 Hz)
4.01 (1H, dd, J = 2.2, 12.4 Hz)
4.15 (2H, m)
4.20 (1H, m)
4.49 (1H, dd, J = 6.5, 7.2 Hz)
6.07 (1H, d, J = 2.5 Hz)
6.60 (1H, t, J = 9.4 Hz)
7.47 (1H, dd, J = 2.5, 9.4 Hz)
8.31 (1H, d, J = 2.5 Hz)
- ¹³C - NMR:: δ (CDCl₃; ppm from TMS);
25.7 (CH₂), 26.2 (CH₃), 29.2 (CH₂), 59.2 (CH₂), 61.0 (CH), 67.9 (CH), 74.9 (CH), 83.8 (CH), 90.1 (CH), 118.7 (C), 119.6 (CH), 131.8 (CH), 138.3 (CH), 162.1 (C), 175.8 (C)
- MS (%):: 325 (M⁺¹) (2), 324 (3), 235 (8), 232 (24), 194 (13), 193 (100), 192 (84), 191 (47), 163 (26), 135 (36), 98 (75), 69 (29), 57 (25)

### Example 8

### Flavor and taste improving agent

The compounds (1a) to (1f), (6e) and (6f) of the present invention, prepared in previous Examples were subjected to the following tests to evaluate their usefulness as a flavor and taste improving agent and a tobacco flavor.
1) The effect of the compounds (1a) to (1f), (6e) and (6f) of the present invention on flavor, taste, and stimulation.
10 % ethanol solutions of the compounds (1a) to (1f), (6e) and (6f) of the present invention were prepared. The ethanol solutions were spray-added to 100 g of shredded tobacco leaf fillings of "MILD SEVEN" (trade name) so as to be contained in an amount of 0.001% (w/w) based on those of the tobacco fillings. The tobacco fillings were rolled up and each was compared to a control sample, namely an unsprayed tobacco filling, in terms of flavor, taste, and stimulation by a discrimination method. The test was performed by 20 especially-trained special panelists.
The results were as shown in the following Tables 1 to 3.
Hereinafter, figures in Tables of the specification indicate the number of people who evaluate excellent and * mark denotes the presence of a significant difference at 5 % danger ratio.

**TABLE 1**

| Comparison Results of Flavor | | |
|---|---|---|
| Compound | Flavored product | Non-flavored product (Control) |
| 1a | 16* | 4 |
| 1b | 17* | 3 |
| 1c | 15* | 5 |
| 1d | 16* | 4 |
| 1e | 15* | 5 |
| 1f | 15* | 5 |
| 6e | 16* | 4 |
| 6f | 16* | 4 |

**TABLE 2**

| Comparison Results of Taste | | |
|---|---|---|
| Compound | Flavored product | Non-flavored product (Control) |
| 1a | 18* | 2 |
| 1b | 17* | 3 |
| 1c | 17* | 3 |
| 1d | 17* | 3 |
| 1e | 18* | 2 |
| 1f | 17* | 3 |
| 6e | 18* | 2 |
| 6f | 17* | 3 |

**TABLE 3**

| Comparison Results of Stimulation | | |
|---|---|---|
| Compound | Flavored product | Non-flavored product (Control) |
| 1a | 13 | 7 |
| 1b | 13 | 7 |
| 1c | 12 | 8 |
| 1d | 12 | 8 |
| 1e | 12 | 8 |
| 1f | 12 | 8 |
| 6e | 12 | 8 |
| 6f | 12 | 8 |

As is apparent from Tables 1 to 3, remarkable improvements in flavor, taste, and stimulation were achieved by addition of the compounds (1a) to (1f), (6e) and (6f) of the present invention.
2) The effect of the compounds (1a) to (1f), (6e) and (6f) of the present invention on flavor in combination with taste
The compounds (1a) to (1f), (6e) and (6f) of the present invention were classified into combinations: (1a) and (1b); (1c) and (1d); (1e) and (6e); and (1f) and (6f), and effects on the improved flavor in combination with taste were tested.

An ethanol solution of the mixture containing compounds (1a) and (1b) of the present invention in a ratio of 1 : 1 was prepared. The solution was spray-added to 100 g of shredded tobacco leaf filling for "CABIN" (trade name) such that it was contained in the shredded tobacco leaf filling in an amount of 0.001% (w/w). The tobacco filling was rolled up and compared to a control sample, namely an unsprayed tobacco filling in terms of flavor in combination with taste at the time of smoking by a discrimination method. The test was performed by 100 people of "CABIN" lovers.

The effects of other combinations on the improvement of flavor and taste were evaluated in a similar fashion.

The results were as shown in the following Table 4.

**TABLE 4**

| Effect on Improving Flavor and Taste | | |
|---|---|---|
| Combination of compounds | Flavored product | Non-flavored product (Control) |
| (1a) and (1b) | 84* | 16 |
| (1c) and (1d) | 82* | 18 |
| (1e) and (6e) | 81* | 19 |
| (1f) and (6f) | 82* | 18 |

As is apparent from Table 4, remarkable improvements on the flavor in combination with taste were attained by addition of the compounds of the present invention.

The compounds (1a) to (1f), (6e) and (6f) of the present invention can be added by methods other than the aforementioned one. To be more specific, the compounds (1a) to (1f), (6e) and (6f) may be used alone or in appropriate mixed form. Further, the compounds (1a) to (1f), (6e) and (6f) of the present invention can be added in an amount of approximately 0.0001 to 0.01% (w/w) based on that of a shredded tobacco filling for use in rolled-up tobacco or a shredded tobacco filling for use in raw material of a tobacco product prior to packaging.

### Industrial Applicability

From the foregoing, the compounds of the present invention are substances excellent as a flavor and taste improving agent and effective in imparting suitable tobacco flavor and taste to a shredded tobacco filling and delicate depth to smoke.

In recent years, the pharmacological activities of and the applications of compounds containing a glycosyl group to pharmaceutical agents have been intensively and extensively studied, producing excellent results. Particularly, a number of attempts have been made to introduce a glycosyl group to a highly toxic compound so as to reduce the toxicity (for example, 5-fluorouracilriboside). Presumably, the compounds having a glycosyl group in a molecule will become important in future. Since the compounds of the present invention have a specific structure containing ribose in a molecule and a nicotine which is physiologically active as an aglycone, the application to pharmaceutical agents is expected other than their application to the abovementioned flavor and taste improving agent.

## Claims

1. A novel nicotine derivative containing a glycosyl group represented by a general formula: 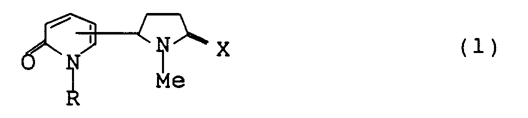 wherein R is a ribofuranosyl group, and X is an oxygen atom or two hydrogen atoms.

2. A novel nicotine derivative containing a glycosyl group according to claim 1, wherein said R and an aglycone are bound with β-N-glycosidic bond.

3. A novel nicotine derivative containing a glycosyl group according to either claim 1 or 2, wherein said aglycone represented by a formula (3) is a (1-methyl-2-pyrrolidinyl)-2-pyridone group or a (1-methyl-5-oxo-2-pyrrolidinyl)-2-pyridone group: 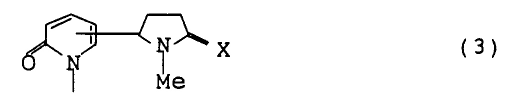 wherein X is the same as defined above.

4. A novel nicotine derivative containing a glycosyl group according to claim 3, wherein an aglycone represented by said general formula (3) is an (S)-(1-methyl-2-pyrrolidinyl)-2-pyridone group, an (R)-(1-methyl-2-pyrrolidinyl)-2-pyridone group, an (S)-(1-methyl-5-oxo-pyrrolidinyl)-2-pyridone group, or an (R)-(1-methyl-5-oxo-2-pyrrolidinyl)-2-pyridone group.

5. A novel nicotine derivative containing a glycosyl group according to claim 3, wherein said aglycone (3) is selected from the group consisting of an (S)-(-)-3-(1-methyl-2-pyrrolidinyl)-2-pyridone group (3a), an (R)-(+)-3-(1-methyl-2-pyrrolidinyl)-2-pyridone group (3b), an (S)-(-)-5-(1-methyl-2-pyrrolidinyl)-2-pyridone group (3c), an (R)-(+)-5-(1-methyl-2-pyrrolidinyl)-2-pyridone group (3d), an (S)-(-)-3-(1-methyl-5-oxo-2-pyrrolidinyl)-2-pyridone group (3e), and an (S)-(-)-5-(1-methyl-5-oxo-2-pyrrolidinyl)-2-pyridone group (3f) shown below.

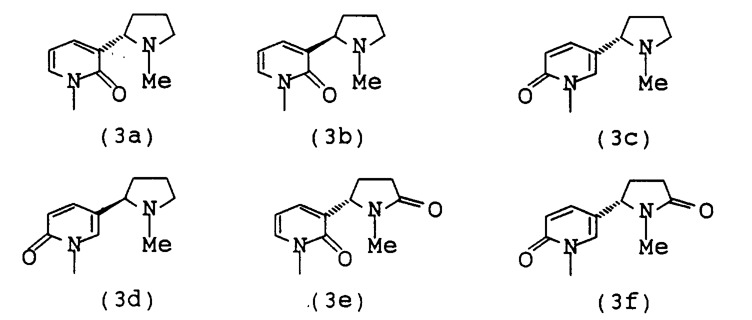

6. A novel nicotine derivative represented by a general formula (2) shown below: 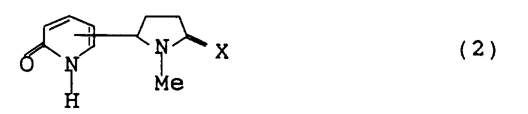 wherein X is the same as defined in claim 1.

7. A novel nicotine derivative according to claim 6, wherein said compound (2) is (1-methyl-2-pyrrolidinyl)-2(1H)-pyridone or (1-methyl-5-oxo-2-pyrrolidinyl)-2(1H)-pyridone.

8. A novel nicotine derivative according to claim 6, wherein said compound (2) is (S)-(1-methyl-2-pyrrolidinyl)-2(1H)-pyridone, (R)-(1-methyl-2-pyrrolidinyl)-2(1H)-pyridone, (S)-(1-methyl-5-oxo-2-pyrrolidinyl)-2(1H)-pyridone, or (R)-(1-methyl-5-oxo-2- pyrrolidinyl)-2(1H)-pyridone.

9. A novel nicotine derivative according to claim 6, said compound (2) is (S)-(-)-3-(1-methyl-2-pyrrolidinyl)-2(1H)-pyridone (2a) represented by a formula shown below:

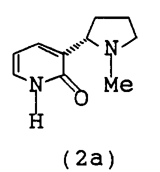

10. A novel nicotine derivative according to claim 6, said compound (2) is (R)-(+)-3-(1-methyl-2-pyrrolidinyl)-2(1H)-pyridone (2b) represented by a formula shown below:

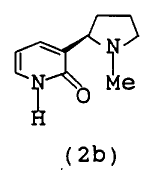

11. A novel nicotine derivative according to claim 6, said compound (2) is (S)-(-)-5-(1-methyl-2-pyrrolidinyl)-2(1H)-pyridone (2c) represented by a formula shown below:

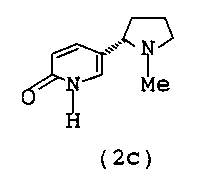

12. A novel nicotine derivative according to claim 6, said compound (2) is (R)-(+)-5-(1-methyl-2-pyrrolidinyl)-2(1H)-pyridone (2d) represented by a formula shown below:

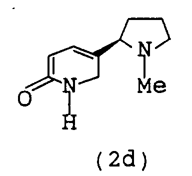

13. A novel nicotine derivative according to claim 6, said compound (2) is (S)-(-)-3-(1-methyl-5-oxo-2-pyrrolidinyl)-2(1H)-pyridone (2e) represented by a formula shown below:

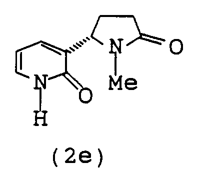

14. A novel nicotine derivative according to claim 6, said compound (2) is (S)-(-)-5-(1-methyl-5-oxo-2-pyrrolidinyl)-2(1H)-pyridone (2f) represented by a formula shown below:
